# EUROPÄISCHE PATENTSCHRIFT

(11) **EP 3 034 108 B1**
(45) Veröffentlichungstag und Bekanntmachung des Hinweises auf die Patenterteilung: **17.10.2018**
(21) Anmeldenummer: 15198614.8
(22) Anmeldetag: 09.12.2015
(51) Int. Cl.: A61M 1/16, A61M 39/10, B01F 15/00

(54) **KONZENTRATBEHÄLTER FÜR EINE EXTRAKORPORALE BLUTBEHANDLUNGSMASCHINE, SOWIE KONZENTRAT-ZUFÜHRSYSTEM FÜR EINE EXTRAKORPORALE BLUTBEHANDLUNGSMASCHINE**
CONCENTRATE CONTAINER FOR AN EXTRACORPOREAL BLOOD TREATMENT MACHINE, AND A CONCENTRATE SUPPLYING SYSTEM FOR EXTRACORPOREAL BLOOD TREATMENT MACHINE
RECIPIENT DE CONCENTRE POUR UNE MACHINE DE TRAITEMENT DE SANG EXTRACORPOREL ET SYSTEME D'ACHEMINEMENT DE CONCENTRE POUR UNE MACHINE DE TRAITEMENT DE SANG EXTRACORPOREL

(30) Priorität: 18.12.2014 DE 102014119106
(43) Veröffentlichungstag der Anmeldung: 22.06.2016
(73) Patentinhaber: B. Braun Avitum AG, 34212 Melsungen (DE)
(72) Erfinder: Ritter, Kai-Uwe, 91126 Rednitzhembach (DE)
(74) Vertreter: Winter, Brandl, Fürniss, Hübner, Röss, Kaiser, Polte - Partnerschaft mbB

(56) Entgegenhaltungen:
- WO-A1-00/44418
- WO-A1-2010/121740
- WO-A1-2012/076287
- DE-U1- 9 302 790
- DE-U1- 20 007 278
- US-B2- 8 182 137

## Beschreibung

Die vorliegende Erfindung betrifft einen Konzentratbehälter einer extrakorporalen Blutbehandlungsmaschine und ein Konzentrat-Zuführsystem einer extrakorporalen Blutbehandlungsmaschine mit einem solchen Konzentratbehälter.

### Technischer Hintergrund der Erfindung

Der Therapieerfolg der Hämodialyse beruht unter anderem auf dem Einsatz verschiedener Puffersubstanzen, so dass der veränderte Säure-Basen-Haushalt der niereninsuffizienten Patienten korrigiert werden kann. Da sich der Säure-Basen-Haushalt nicht während der Dialyse durch Diffusion oder Konvektion korrigieren lässt, ist die Zufuhr von Puffersubstanzen unabdingbar. Theoretisch eignen sich Bicarbonat, Acetat und Laktat zum Ausgleich des Gefälles zwischen Säuren und Basen, allerdings erfolgt die Hämodialysebehandlung auf Grund diverser Nachteile der Laktat- und Acetatpufferung meist ausschließlich unter Verwendung der Bicarbonatpufferung. Die Puffersubstanz ist der wichtigste Bestandteil bei einer Dialysierflüssigkeit.

Um das Gewicht der Dialysemaschine zu verringern, sowie um den für die Lagerung der Bikarbonatkartuschen benötigten Stauraum zu reduzieren und etwaigen Kontaminationen des Bikarbonats vorzubeugen, wird die Bicarbonat- bzw. Pufferlösung für ein medizinisches Verfahren wie beispielsweise eine Hämodialyse erst unmittelbar vor bzw. während der Behandlung erzeugt.

Zu diesem Zweck wird herkömmlicherweise ein Behälter wie beispielsweise eine Kartusche oder Patrone, welcher ein Bicarbonatkonzentratpulver enthält, an eine Fluidquelle wie z.B. eine Wasserquelle angeschlossen. Das die Kartusche durchströmende Wasser löst das darin gespeicherte Bicarbonatkonzentratpulver und schwemmt dieses in dosierter Weise in die Dialysierflüssigkeit aus.

### Stand der Technik

Derartige herkömmliche Konzentratkartuschen, wie sie allgemein aus dem Stand der Technik bekannt sind, haben in der Regel einen Patronen- oder zylinderförmigen Aufnahmebehälter, an dessen einer axialer Stirnseite bzw. an dessen einem axialen Ende ein Einlass-Konnektor zum Verbinden der Kartusche mit einer Fluideinlassleitung und an dessen anderer axialer Stirnseite bzw. einem anderen Ende ein Auslass-Konnektor zum Verbinden der Kartusche mit einer Fluidauslassleitung angeordnet/ausgebildet ist. Diese Konnektoren einer herkömmlichen Konzentratkartusche bestehen aus kurzen, von der jeweiligen Kartusche bzw. den Aufnahmebehälter (axial) hervorragenden Rohrabschnitten oder Rohrstutzen, deren Innendurchmesser größer ist als der Außendurchmesser der Fluideinlassleitung bzw. der Fluidauslassleitung, sodass jeweils eine Fluidleitung in einen von der Kartusche/Aufnahmebehälter hervorragenden stutzenförmigen Konnektor eingesteckt werden kann. Derartige Konzentratkartuschen mit axial nach außen hervorragenden Rohrstutzen sind aus dem Stand der Technik beispielsweise aus den Druckschriften WO 2012/076287 A1, DE 200 07 278 U1, DE 93 02 790 U1 oder WO 00/44418 A1 bekannt.

Derartige, herkömmlicherweise stutzenförmige Konnektoren bringen jedoch den Nachteil mit sich, dass sie beim Transport der Konzentratkartuschen leicht beschädigt werden können (beispielsweise können die hervorstehenden Konnektoren abknicken/abbrechen), wodurch die jeweilige Kartusche unbrauchbar wird. Zudem erhöht sich der Platzbedarf der Kartuschen im Transportbehälter, wodurch sich die Logistikkosten der Kartuschen unnötig erhöhen. Auch die Materialkosten bei der Fertigung herkömmlicher Kartuschen sind unnötig hoch. Schließlich müssen die stutzenförmigen Konnektoren aufwändig in Schutzmaterial verpackt werden, um sie vor Beschädigung insbesondere beim Transport zu bewahren.

Ein weiterer Nachteil herkömmlicher Konzentratkartuschen besteht darin, dass die Verteilung des über den Einlass-Konnektor in den Aufnahmebehälter einströmenden Fluids auf das Konzentratpulver nur ungenügend erfolgt. Durch die axial zentrale Einleitung des Fluids in die Konzentratkartusche/Aufnahmebehälter, kann es in dem darin enthaltenen Konzentratpulver zu einer axial zentralen Kanalbildung entlang der Fluidflussrichtung kommen. Dies führt dazu, dass das Konzentrat nicht optimal genutzt wird, da das Fluid hauptsächlich durch den gebildeten Kanal strömt und das an den Seitenwänden der Kartusche angelagerte Konzentrat nicht (vollständig) gelöst wird. Somit verringert eine solche Kanalbildung die Effizienz der Kartusche und erschwert es zudem, die Konzentration des gelösten Stoffes in der Pufferlösung akkurat zu steuern. Auch besteht durch die Kanalbildung besonders bei Dialysemaschinen, die in der Regel einen Unterdruck in der Kartusche erzeugen, die Gefahr, dass die Kartuschen kollabieren. Erste Ansätze, Fluidlenkungsmittel einzusetzen, welche eine derartige Kanalbildung verringern können, sind im Stand der Technik beispielsweise in den Druckschriften WO 2010/121740 A1 oder US 8 182 137 B2 offenbart.

Aufgrund der unvorteilhaften Auslegung herkömmlicher Kartuschen ist auch der Desinfektionsvorgang des gesamten Systems zur Herstellung eines Fließmittels (Konzentrat-Zuführsystem) nach dessen Verwendung sehr aufwändig. Bei einem bekannten System zur Herstellung von Fließmitteln wie z.B. Pufferlösungen sind beispielsweise Kartuschenhalter mit Klapparmen an der Außenseite einer Dialysemaschine vorgesehen. Bei der Desinfektion dieses Systems bzw. der Dialysemaschine werden die Klapparme in Richtung hin zur Maschinenwand eingeklappt und die Anschlussstücke ruhen abgedichtet in Öffnungen innerhalb der Maschinenwand, welche Maschineninnenseitig durch ein internes Verbindungsstück kurzgeschlossen sind.

Nachdem die Klapparme auf diese Weise eingeklappt sind, kann das gesamte (Leitungs-) System zur Desinfektion durchgespült werden. Durch die Notwendigkeit des Einklappens der Klapparme ist dieser Vorgang unnötig zeitaufwendig und zudem führt diese Ausgestaltung zu Hinterschneidungen zwischen den Klapparmen und den Kartuschenhaltern, die die Reinigung des Systems erschweren.

Alternativ wird bei einem anderen, aus dem Stand der Technik bekannten, System zur Herstellung eines Fließmittels ein externes Verbindungsstück zwischen den oberen und unteren Halter der Kartusche eingesetzt. Dieses Verbindungsstück fährt in Öffnungen in den Haltern ein, welche eigentlich zur Aufnahme der stutzenförmigen Einlass-/Auslass-Konnektoren der Kartusche dienen und wird radial von außen abgedichtet. Dieses Einsetzen des externen Verbindungsstücks in die entsprechenden Öffnungen der Halter verkompliziert das Einbringen des Verbindungsstücks. Zudem vergrößert die Anordnung der radialen Abdichtung an der Außenseite des Verbindungsstücks den Umfang des Verbindungsstücks unnötig.

### Zusammenfassung der Erfindung

Ausgehend von dem oben genannten Stand der Technik liegt der vorliegenden Erfindung die Aufgabe zugrunde, einerseits einen preiswerten Konzentratbehälter/Kartusche bereitzustellen, bei dem das durchströmende Fluid (Wasser) optimal auf das Konzentrat verteilt wird und andererseits den Desinfektionsvorgang eines Systems zur Herstellung eines Fließmittels (auch als Konzentrat-Zuführsystem bezeichnet) nach der Behandlung zu vereinfachen.

Die Lösung dieser Aufgabe erfolgt durch den Konzentratbehälter nach Anspruch 1 und das System für eine extrakorporale Blutbehandlungsmaschine zur Herstellung eines Fließmittels nach Anspruch 9. Vorteilhafte Weiterbildungen und Abwandlungen der Erfindung sind Gegenstand der Unteransprüche.

Der Kerngedanke der Erfindung liegt demzufolge darin, die Anschlussstück-Konnektor-Kopplung zwischen einem Konzentratbehälter (bzw. Kartusche) und den daran anzuschließenden Fluideinlass- und Fluidauslassleitungen nach dem Stecker-Steckdose-Prinzip auszubilden, wobei die Steckdose in Form des Konnektors auf Seiten der Kartusche und der Stecker in Form des Anschlussstücks auf Seiten der Leitungen angeordnet ist. Dabei ist der Kartuschenseitige Konnektor nach Art einer Unterputz-Steckdose ausgebildet, die nicht oder nur geringfügig über die Wandoberfläche vorragt.

In anderen Worten ausgedrückt wird das jeweilige Anschlusstück einer jeweiligen Fluideinlass- oder Fluidauslassleitung nach Art eines Steckers ausgebildet, d.h. als hervorstehendes/hervorragendes bzw. konvexes Bauteil. Der jeweilige Konnektor des Konzentratbehälters/Kartusche, der der Aufnahme eines jeweiligen Anschlusstücks einer Fluidleitung dient, ist nach Art einer Steckdose ausgebildet, d.h. als vollständig innerhalb des Konzentratbehälters/Aufnahmebehälters der Kartusche angeordnetes, in den Konzentratbehälter/Aufnahmebehälter der Kartusche zurückweichendes /eingebuchtetes bzw. konkaves Bauteil.

Zum Anschließen des Konzentratbehälters/Kartusche beispielsweise an eine extrakorporale Blutbehandlungsmaschine zur Herstellung eines Fließmittels wie beispielsweise einer Bicarbonatpufferlösung werden die konvexen / hervorstehenden Anschlussstücke der Fluideinlass- und Fluidauslassleitungen der extrakorporalen Blutbehandlungsmaschine in die entsprechenden konkaven / in den jeweiligen Konzentratbehälter/Aufnahmebehälter der Kartusche zurückweichenden Konnektoren eingesteckt und darin zumindest abschnittsweise oder sogar vollständig aufgenommen.

Dies hat den Vorteil, dass die hervorstehenden / konvexen Anschlussstücke maschinenseitig auf Seiten der Fluideinlass- und Fluidauslassleitungen der Blutbehandlungsmaschine angeordnet sind und nicht, wie aus dem Stand der Technik bekannt, Teil des Konzentratbehälters/Kartusche sind. Dadurch, dass der Konzentratbehälter/Kartusche keine oder nur geringfügig von der Behälterwand vorragenden Teile (Konnektoren) hat, verringert sich der für den Transport und die Lagerung der Konzentratbehälter/Kartuschen benötigte Stauraum und die Gefahr der Beschädigung der Konzentratbehälter/Kartuschen wird reduziert. Auch werden die Materialkosten bei der Herstellung der Konzentratbehälter/Kartuschen reduziert und Verpackungsmaterial eingespart. Zudem wird das Einsetzen der Konzentratbehälter in das System zur Herstellung eines Fließmittels wie beispielsweise einer Bicarbonatpufferlösung erleichtert, da keine hervorstehenden Kleinteile wie beispielsweise die aus dem Stand der Technik bekannten Konnektoren in Form von, von den Konzentratbehältern/Aufnahmebehältern hervorstehenden stutzenförmigen Rohrabschnitten abgeknickt oder sonstwie beschädigt werden können.

In einer vorteilhaften Ausführungsform weist ein erfindungsgemäßer Konzentratbehälter/Kartusche an einem ersten axialen Ende (axiale Stirnseite des Aufnahmebehälters der Kartusche) einen Fluideinlass auf, das heißt, eine Öffnung, durch die Fluid (Wasser) in den Aufnahmebehälter fließen kann. Der Fluideinlass ist, wie vorstehend bereits angedeutet, als ein in den Aufnahmebehälter der Kartusche hineinragender, konkaver Konnektor zur Aufnahme eines Anschlussstücks einer Fluidleitung ausgebildet. An einem zweiten Ende (axiale Stirnseite des Aufnahmebehälters der Kartusche) weist der erfindungsgemäße Konzentratbehälter/Kartusche einen Fluidauslass auf, das heißt, eine Öffnung, durch die Fluid aus dem Behälter fließen kann. Der Fluidauslass ist vorzugsweise ebenfalls als ein in den Aufnahmebehälter der Kartusche hineinragender, konkaver Konnektor zur Aufnahme eines Anschlussstücks einer Fluidleitung angeordnet.

Durch die Fluideinlassleitung fließt ein Fluid wie beispielsweise Wasser in den Konzentratbehälter/Aufnahmebehälter der Kartusche und löst das Konzentrat im Inneren des Konzentratbehälters/Aufnahmebehälters. Über die Fluidauslassleitung fließt die Lösung aus Konzentrat und beispielsweise Wasser aus dem Konzentrat-/Aufnahmebehälter hinaus und kann beispielsweise im Rahmen einer extrakorporalen Blutbehandlung einem Patienten zugeführt werden.

Um zu gewährleisten, dass möglichst die gesamte Lösung aus dem Konzentratbehälter/Kartusche fließt und möglichst wenig Lösung in dem Konzentratbehälter/Kartusche zurückbleibt, ist in einer vorteilhaften Ausführungsform das zweite Ende (axiale Stirnseite) des Konzentrat-/Aufnahmebehälters, d.h. das axiale Ende des Konzentrat-/Aufnahmebehälters der Kartusche, welches mit der Fluidauslassleitung verbunden/verbindbar ist, in Form eines Kugelsegments (vorzugsweise Halbkugel) oder eines Kegels ausgebildet. Aufgrund dieser geometrischen Ausbildung sammelt sich die Lösung am tiefsten Punkt des Kugelsegments bzw. des Kegels. Wird auch der Fluidauslass des Behälters an diesem tiefsten Punkt angeordnet, so bleibt möglichst wenig Lösung in dem Konzentrat-/Aufnahmebehälter zurück. Durch diese Ausgestaltung des Konzentratbehälters/Kartusche erhöht sich die Effizienz des Konzentratbehälters/Kartusche.

Um die Effizienz des Konzentratbehälters/Kartusche durch optimale Verwendung des Konzentrats weiter zu verbessern, weist der Konzentratbehälter/Kartusche in einer weiteren vorteilhaften Ausführungsform zusätzlich ein Fluidlenkungsmittel (Stromleitelement) auf, welches in Fluidströmungsrichtung gesehen mindestens einem Konnektor zur Aufnahme eines Anschlussstücks einer Fluidleitung vorzugsweise zum Fluideinlass in den Behälter nachgeschaltet ist. Dieses Fluidlenkungsmittel dient dazu, den Fluidfluss zu lenken und zu regulieren, um so einer Kanalbildung in dem Konzentratpulver entgegenzuwirken. Beispielsweise spaltet des Fluidlenkungsmittel den Fluidstrom, welcher in den Konzentrat-/Aufnahmebehälter einströmt, in mehrere kleinere Teilströme auf, die auf das Konzentrat verteilt werden und/oder das Fluidlenkungsmittel verlangsamt den Fluidfluss, so dass mehr Konzentrat gelöst wird. Wird die Kanalbildung in dem Konzentrat-/Aufnahmebehälter auf diese Weise unterbunden, so verringert sich auch die Gefahr des Kollabierens des Konzentratbehälters/Kartusche insbesondere bei Verwendung mit einer Blutbehandlungsmaschine, die einen Unterdruck in dem Konzentratbehälter/Kartusche erzeugt, da keine unkontrollierten Hohlräume wie beispielsweise ein zentraler Kanal in dem Konzentrat entstehen.

In einer vorteilhaften Ausgestaltung bilden das Fluidlenkungsmittel und der Konnektor, welchem das Fluidlenkungsmittel in Fluidströmungsrichtung nachgeschaltet ist, ein integrales Bauteil. Dies vereinfacht die Herstellung des Konzentratbehälters/Kartusche und erhöht zudem die Haltbarkeit und Belastbarkeit der Verbindung zwischen dem Fluidlenkungsmittel und dem Konnektor. Beispielsweise ist das Bauteil aus Fluidlenkungsmittel und Konnektor einstückig im Spritzgussverfahren (vorzugsweise aus Kunststoff) oder auch als Blechbiegeteil gefertigt.

In seiner unkompliziertesten Ausführungsform ist das Fluidlenkungsmittel eine Aufprallscheibe (Sprinklerkopfscheibe), auf die in den Behälter einströmendes Fluid aufprallt, wodurch das Fluid vorzugsweise gleichmäßig auf das in dem Behälter enthaltene Konzentrat verteilt wird. Eine solche Aufprallscheibe kann an ihrer der Fluidströmung zugewandten Oberfläche glatt ausgestaltet sein, allerdings auch Strukturen wie beispielsweise Rillen oder Finnen zur Lenkung des Fluidstroms aufweisen.

Weiterhin kann das Fluidlenkungsmittel auch drehbar, vorzugsweise über ein turbinenartiges Flügelrad, gelagert sein. Durch die Drehung des Fluidlenkungsmittels, wie beispielsweise der Aufprallscheibe, wird die Fluidströmung weiter vorzugsweise in mehrere Teilströme aufgespalten und zudem in radialer Richtung abgelenkt, so dass das Fluid auch Konzentrat an den Seitenwänden des Konzentratbehälters benetzt. So wird eine optimale und gleichmäßige Verwendung des Konzentrats ermöglicht und einer Kanalbildung im Konzentrat entgegengewirkt. Anstatt durch eine drehbare Lagerung des Fluidlenkungsmittels kann die radiale Ablenkung der Fluidströme alternativ auch durch die Anordnung der Strukturen wie beispielsweise Rillen oder Finnen auf der Aufprallscheibe erfolgen. So können die Strukturen beispielsweise in radialer Richtung gekrümmt/turbinenschaufelartig gebogen sein.

Soll die Fluidströmung nur verlangsamt, allerdings nicht radial abgelenkt werden, so kann in einer weiteren Ausführungsform das Fluidlenkungsmittel als Sieb ausgebildet werden. Durch Variieren der Maschenweite des Siebes kann die Flussgeschwindigkeit des Fluids eingestellt werden.

Maschinenseitig weist ein (Leitungs-)System für eine extrakorporale Blutbehandlungsmaschine zur Herstellung beispielsweise einer Pufferlösung, welches in Verbindung mit einem oben beschriebenen Konzentratbehälter/Kartusche verwendet werden soll, mindestens ein Anschlussstück zum Fluidverbinden des Konzentratbehälters/Kartusche mit einer Fluideinlassleitung und mindestens ein Anschlussstück zum Fluidverbinden des Konzentratbehälters/Kartusche mit einer Fluidauslassleitung auf. Hierbei ist mindestens eines der Anschlussstücke, jedoch vorzugsweise beide Anschlussstücke, konvex (vorzugsweise Stöpsel-/Stopfen-förmig) ausgebildet und dazu ausgelegt, in einen entsprechenden konkaven und innerhalb des Konzentrat-/Aufnahmebehälters angeordneten Konnektor des Konzentratbehälters/Kartusche einzugreifen bzw. nach dem Stecker-Steckdose-Prinzip eingesteckt zu werden.

Da bei diesem System die konvexen, d.h. hervorstehenden Anschlussstücke, in anderen Worten die Stecker, maschinenseitig als Teil der Blutbehandlungsmaschine bereitgestellt werden, entfällt bei diesem System die Notwendigkeit, die Konzentratbehälter/Kartuschen mit hervorstehenden, leicht zu beschädigenden Verbindungsstücken auszustatten, wie dies im Stand der Technik der Fall ist. Stattdessen liegen die Konnektoren der erfindungsgemäßen Konzentratbehälter/Kartuschen innerhalb des Aufnahmebehälters der jeweiligen Kartuschen, sind somit konkav ausgebildet und entsprechen somit in der Stecker-Steckdose-Analogie der (Unterputz-)Steckdose. Da die Blutbehandlungsmaschine im Gegensatz zu den Konzentratbehältern/Kartuschen kein Einmalartikel ist, können die konvexen Anschlussstücke der Fluidleitungen der Blutbehandlungsmaschine robuster ausgebildet werden (es besteht weniger Druck, die Materialkosten zu senken), so dass kein Risiko besteht, dass diese Anschlussstücke beim Einsetzen des Konzentratbehälters abknicken.

Zudem wird das Einsetzen des Konzentratbehälters in die Blutbehandlungsmaschine durch das erfindungsgemäße System, bei dem die Anschlussstücke der Fluidleitungen der Blutbehandlungsmaschine und die Konnektoren der Konzentratbehälter/Kartuschen nach dem Stecker- Steckdose-Prinzip konstruiert sind, stark vereinfacht. Dies ist besonders im hektischen klinischen Alltag eine große Erleichterung.

Zudem ist auch die Desinfektion bzw. die Reinigung und/oder Spülung eines (Leitungs-) Systems, bei dem die Anschlussstücke der Fluidleitungen der Blutbehandlungsmaschine und die Konnektoren der Konzentratbehälter/Kartuschen nach dem Stecker- Steckdose-Prinzip konstruiert sind, vereinfacht.

Nach der Blutbehandlung unter Verwendung beispielsweise eines Konzentratbehälters/Kartusche mit Bicarbonatkonzentrat wird vorzugsweise der Konzentratbehälter/Kartusche aus der Blutbehandlungsmaschine entfernt und die Blutbehandlungsmaschine wird gereinigt. Hierbei kann für die Desinfektion des Systems ein (externes) Verbindungsstück zum Verbinden der Anschlusstücke der Fluideinlassleitung und Fluidauslassleitung miteinander bereitgestellt sein, welches bevorzugt die Form eines Hohlzylinders hat, dessen Innendurchmesser wenigstens im Bereich seiner beiden axialen Endabschnitte dem Außendurchmesser mindestens eines Anschlussstücks einer Fluidleitung des Systems entspricht und damit die Konnektoren der bereits entnommenen Kartusche simuliert. Dieses Verbindungsstück wird während des Desinfektions- oder Spülvorgangs an Stelle des Konzentratbehälters/Kartusche auf die Anschlussstücke der Blutbehandlungsmaschine aufgesteckt. Dadurch sind die Fluideinlassleitung und die Fluidauslassleitung über das Verbindungsstück fluidisch verbunden (kurzgeschlossen) und die Blutbehandlungsmaschine kann durchgespült werden.

Aufgrund der vereinfachten Ausgestaltung der Verbindung/Kopplung zwischen den konvexen Anschlussstücken der Leitungen der Blutbehandlungsmaschine und den konkaven (in den Aufnahmebehälter der Kartusche hinragenden) Konnektoren des Konzentratbehälters/Kartusche nach dem Stecker-Steckdose-Prinzip läßt sich auch das Verbindungsstück sehr einfach auf die Anschlussstücke der Blutbehandlungsmaschine aufstecken. Das Verbindungsstück ersetzt hierbei strukturell den Konzentratbehälter/Kartusche und die Öffnungen des Hohlzylinders des Verbindungsstücks stellen die "Steckdosen" (Konnektoren) dar, in die die "Stecker" (Anschlussstücke) der Leitungen jeweils eingesteckt werden.

Durch diese Ausgestaltung der Verbindung/Kopplung zwischen den Anschlussstücken der Leitungen der Blutbehandlungsmaschine und dem (externen) Verbindungsstück wird der Reinigungsvorgang der Blutbehandlungsmaschine stark vereinfacht, da beispielsweise keinerlei Klapparme vor der Reinigung eingeklappt werden müssen oder das Verbindungsstück in Aussparungen in den Kartuschen-Haltearmen präzise eingefädelt werden muss. Hierdurch wird der Reinigungsvorgang beschleunigt und benutzerfreundlicher gemacht.

Das gegenüber dem Stand der Technik vereinfachte Verfahren zur Desinfektion eines erfindungsgemäßen Systems zur Herstellung beispielsweise einer Pufferlösung, bei dem die Anschlussstücke der Fluidleitungen der Blutbehandlungsmaschine und die Konnektoren der Konzentratbehälter/Kartuschen nach dem Stecker- Steckdose-Prinzip konstruiert sind, umfasst somit
- einen ersten Schritt, in dem ein oben genanntes Verbindungsstück an seinen Konnektor simulierenden axialen Enden auf mindestens ein Anschlussstück des Leitungs-Systems aufgesteckt wird und
- einen zweiten Schritt, in dem ein Spülvorgang des Systems durchgeführt wird.

Das Verbindungsstück kann hierbei auf nur ein Anschlussstück des Leitungs-Systems aufgesteckt werden, wenn der dem Konzentratbehälter vorgeschaltete Anteil des Systems beispielsweise mit einer anderen Lösung gespült werden soll, als der dem Konzentratbehälter nachgelagerte Teil des Systems. So wird beispielsweise nur das Anschlussstück der Fluideinlassleitung in das Verbindungsstück eingesteckt, wenn der in Fluidrichtung dem Konzentratbehälter vorgeschaltete Anteil des (Leitungs-) Systems mit einer anderen Lösung gespült werden soll, als der dem Konzentratbehälter/ Kartusche nachgelagerte Teil des Systems. In einem weiteren Schritt kann das externe Verbindungsstück mit dem Anschlussstück der Fluidauslassleitung verbunden werden und der dem Konzentratbehälter/Kartusche nachgelagerte Teil des Systems separat gespült werden.

Weitere Vorteile und Merkmale der vorliegenden Erfindung ergeben sich aus der folgenden Beschreibung bevorzugter Ausführungsbeispiele mit Bezug auf die zugehörigen Figuren.

Es zeigt
Fig. 1 die Prinzipskizze eines erfindungsgemäßen Konzentratbehälters/Kartusche gemäß einer bevorzugten Ausführungsform der Erfindung,
Fig. 1a die Prinzipskizze eines Fluidlenkungsmittels in Form einer Aufprallscheibe,
Fig. 1b eine kegelförmige Ausgestaltung eines den Fluidauslass aufweisenden Endes eines erfindungsgemäßen Konzentratbehälters/Kartusche,
Fig. 2 ein erfindungsgemäßes (Leitungs-)System für eine extrakorporale Blutbehandlungsmaschine zur Herstellung beispielsweise einer Pufferlösung mit einem erfindungsgemäßen Konzentratbehälter/Kartusche und
Fig. 3 ein externes Verbindungsstück, welches in ein erfindungsgemäßes (Leitungs-)System für eine extrakorporale Blutbehandlungsmaschine zur Herstellung beispielsweise einer Pufferlösung anstelle einer erfindungsgemäßen Kartusche insbesondere zu Reinigungs-/Desinfektionszwecken eingesetzt/einsetzbar ist.
Bei der Beschreibung der Figuren bezeichnen die gleichen Bezugszeichen in den Figuren gleiche oder ähnliche Bauteile.

### Figurenbeschreibung

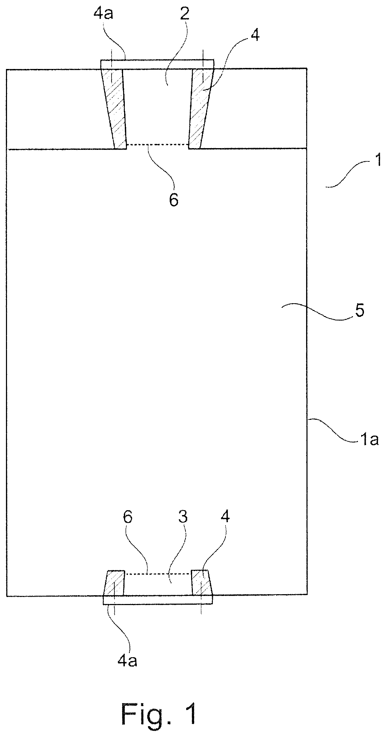

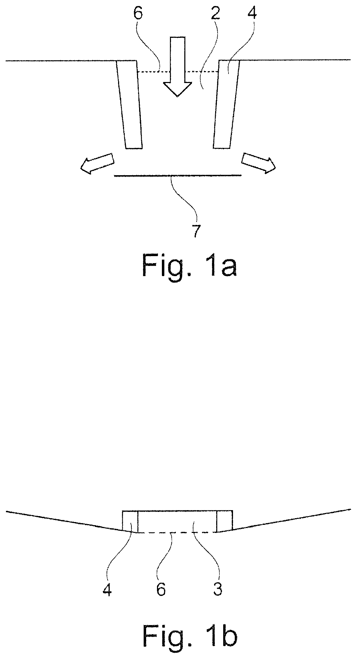

Fig. 1 zeigt einen Längsschnitt durch einen erfindungsgemäßen Konzentratbehälter/Kartusche 1 für eine (einer) extrakorporale(n) Blutbehandlungsmaschine (nicht weiter dargestellt), mit einem Fluideinlass 2 an einem ersten Ende (erste axiale Stirnseite) des Konzentratbehälters/zylinderförmige Kartusche 1 und einem Fluidauslass 3 an einem zweiten Ende (zweite axiale Stirnseite) des Konzentratbehälters/Kartusche 1. Der Konzentratbehälter/Kartusche 1 hat ferner einen im Wesentlichen als Hohlzylinder ausgeformten Aufnahmebehälter 1a zur Einlagerung von Konzentrat, an dem axialbeabstandet der Fluideinlass 2 und der Fluidauslass 3 angeordnet sind. Sowohl der Fluideinlass 2 als auch der Fluidauslass 3 ist jeweils in Form eines buchsenartigen Konnektors 4 zur Aufnahme jeweils eines Anschlussstücks 8, 9 einer Einlass- und Auslass-Fluidleitung (nicht weiter dargestellt) beispielsweise einer Blutbehandlungsmaschine ausgebildet. Die Konnektoren 4 sind konkav ausgebildet und vollständig innerhalb des Aufnahmebehälters 1a angeordnet, wobei der äußere Umfang des Konzentratbehälters/Aufnahmebehälters 1a nicht oder nur geringfügig durch ein Hervorstehen eines Konnektors 4 vergrößert wird. Das Innnere des Konzentratbehälters/Aufnahmebehälters 1a ist mit einem Konzentrat 5, vorzugsweise in Form eines Pulvers, gefüllt. Besonders bevorzugt ist es, dass das Konzentrat 5 Bicarbonatpulver ist.

Zur Gewährleistung der Sterilität des Konzentrats 5 vor der Verwendung des Konzentratbehälters/Kartusche 1 ist sowohl der Fluideinlass 2 (d.h. die Öffnung, durch die Fluid bei der Verwendung des Konzentratbehälters/Kartusche 1 in den Konzentratbehälter/Aufnahmebehälter 1a einströmt), als auch der Fluidauslass 3 (d.h. die Öffnung, durch die Fluid bei der Verwendung des Konzentratbehälters/Kartusche 1 aus dem Konzentratbehälter/Aufnahmebehälter 1a herausströmt) jeweils mit einer Membran 6 versiegelt.

Wird der Konzentratbehälter/Kartusche 1 zur Herstellung beispielsweise einer Bicarbonatpufferlösung im Rahmen einer Blutbehandlung an einer extrakorporalen Blutbehandlungsmaschine (Dialysemaschine) verwendet, so wird das stopfenartige Anschlussstück 8 einer Fluidzuführleitung, welche Fluid dem Konzentratbehälter/ Kartusche 1 zuführt, in den buchsenartigen Konnektor 4 an dem Fluideinlass 2 des Konzentratbehälters 1 eingesteckt, wobei auch die den Aufnahmebehälter 1a versiegelnde Membran 6 durchstoßen wird. Zudem wird in den Konnektor 4 an dem Fluidauslass 3 der Kartusche 1 ein Anschlussstück 9 einer Fluidableitleitung eingesteckt, die Fluid aus dem Konzentratbehälter/Kartusche 1 ableitet und die ebenfalls die Membran 6 durchstößt.

Wird im Konkreten ein solches Anschlussstück 8, 9 in einen der beiden Konnektoren 4 des Konzentratbehälters/Kartusche 1 eingesteckt, so wird das Siegel der Membran 6 beim Einsteckvorgang zwangsläufig gebrochen und Fluid kann über das Konzentrat 5 im Inneren des Konzentratbehälters/Aufnahmebehälters 1a fließen und dieses Konzentrat 5 lösen. Um ein ungewolltes Durchbrechen der Membranen 6 zu Verhindern, kann der Konzentratbehälter/Kartusche 1 jeweils an seinem Fluideinlass 2 und /oder seinem Fluidauslass 3 einen separaten, manuell abnehmbaren Verschlußdeckel 4a aufweisen, mit dem der Fluideinlass 2 und /oder der Fluidauslass 3 vor, die Membran 6 ungewollt punktierenden Teilen geschützt wird.

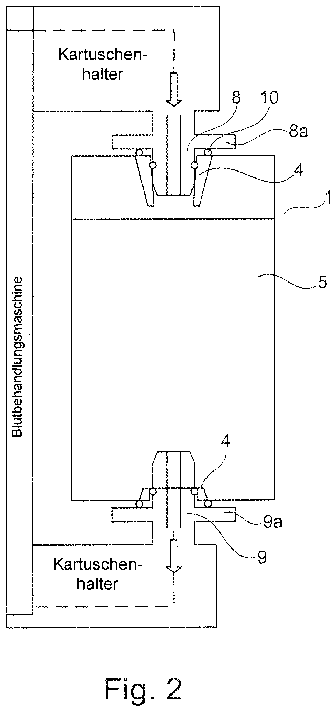

Fig. 1a zeigt eine Detailansicht eines Fluidlenkungsmittels in Form einer Aufprallscheibe 7, die einem Konnektor 4 vorzugsweise auf Seiten des Fluideinlasses in Fluidflussrichtung (dickgedruckter Pfeil in Fig. 2) nachgeschaltet ist. Der Konzentratbehälter/Kartusche 1 aus Fig. 1a ist in einem Zustand dargestellt, in welchem er noch nicht an Fluidleitungen angeschlossen war, weswegen die Membran 6 am Ein- und Auslass noch intakt ist und den Fluideinlass 2 und/oder Fluidauslass 3 des Konzentratbehälters/Kartusche 1 versiegelt. Wird jedoch das Anschlusssstück 8 einer Fluidzuführleitung in den Konnektor 4 des Fluideinlasses 2 des Konzentratbehälters/Kartusche 1 eingesteckt, so wird die zugehörige Membran 6 durch das Anschlusssstück 8 durchbrochen und Fluid fließt in Fluidflussrichtung (dickgedruckter Pfeil) in den Aufnahmebehälter 1a der Kartusche 1. Direkt nach dem Fluideinlass 2 prallt das Fluid jedoch auf die Aufprallscheibe 7, wodurch die Fluidströmung verlangsamt wird und/oder in mehrere Teilströmungen aufgespalten wird, die jeweils radial nach außen (angezeigt durch die beiden dünn gedruckten, kleineren Pfeile) von der ursprünglichen Fluidflussrichtung (dickgedruckter Pfeil) abgelenkt werden. Durch diese Aufprallscheibe 7 wird der unkontrollierten (axialen) Kanalbildung in dem Konzentrat 5 aufgrund eines steten axial zentralen Fluidflusses durch das Konzentrat 5 entlang der Fluidflussrichtung (dickgedruckter Pfeil) vorgebeugt. Auch die Effizienz des Konzentratbehälters/Kartusche 1 vergrößert sich, da beispielsweise auch an den Seitenwänden des Hohlzylinders des Konzentratbehälters/Aufnahmebehälters 1 anliegendes Konzentrat 5, das heißt von der ursprünglichen Fluidflussrichtung (dickgedruckter Pfeil) radial außenliegendes Konzentrat 5 von dem Fluid benetzt wird.

Fig. 1b zeigt eine Detailansicht eines Konzentratbehälters/Kartusche 1, bei dem das axiale Ende (Stirnseite) des Konzentratbehälters 1 mit dem Fluidauslass 3 kegelstumpfförmig bzw. nach Außen gewölbt ausgestaltet ist. Diese geometrische Ausgestaltung des Konzentratbehälters 1 bewirkt ein möglichst restloses Ausfließen von Fluid aus dem Konzentratbehälter 1. Der Fluidauslass 3 ist hierfür am tiefsten Punkt des kegelstompfförmigen/gewölbten Bodens des Konzentratbehälters/Kartusche angeordnet. Auch diese Detailansicht zeigt einen Konzentratbehälter/Kartusche 1 vor dessen Verwendung, so dass die Membranen 6 noch intakt sind.

Fig. 2 zeigt ein erfindungsgemäßes System für eine extrakorporale Blutbehandlungsmaschine (Dialysemaschine) zur Herstellung beispielsweise einer Pufferlösung mit einem erfindungsgemäßen Konzentratbehälter/Kartusche 1. Neben dem erfindungsgemäßen Konzentratbehälter/Kartusche 1 weist dieses System das Anschlussstück 8 zum Fluidverbinden des Konzentratbehälters/Kartusche 1 mit der Fluideinlassleitung bzw. Fluidzuführleitung und mindestens das Anschlussstück 9 zum Fluidverbinden des Konzentratbehälters/Kartusche 1 mit der Fluidauslassleitung bzw. Fluidableitleitung auf. Bei diesem System sind die beiden Anschlussstücke 8 und 9 konvex ausgebildet, d.h. schaftartig als von den Leitungsenden vorzugsweise axial vorstehende Bauteile jeweils nach Art eines Steckers, Stöpsels oder Stopfens ausgelegt. Daher sind die beiden Anschlusstücke 8 und 9 dazu ausgelegt, jeweils in den entsprechend konkaven und innerhalb des Konzentratbehälters 1 angeordneten sowie nach Art einer Steckdose buchsenartig in den Aufnahmebhälter 1a der Kartusche 1 hineinragenden Konnektor 4 des Konzentratbehälters/Katusche 1 einzugreifen bzw. eingesteckt zu werden.

Wie in Fig. 2 gezeigt, sind die beiden Anschlussstücke 8 und 9 in die beiden Konnektoren 4 eingesteckt, die Membranen 6 (nicht gezeigt) sind aufgebrochen und Fluid kann in Fluidflussrichtung (dickgedruckter Pfeil) durch den Konzentrat-/ Aufnahmebehälter 1a fließen. Zur Abdichtung der Steckverbindung eines jeweiligen Anschlussstücks 8, 9 und eines Konnektors 4 werden Dichtungsmittel, beispielsweise O-Ringe 10, verwendet. Hierbei erfolgt die Abdichtung vorzugsweise über zwei O-Ringe 10 pro Verbindung/Kopplung zwischen dem Anschlussstück 8, 9 und dem Konnektor 4. Ein O-Ring 10 ist innerhalb des Konzentrat-/Aufnahmebehälters 1 in der Öffnung des Fluideinlasses 2 oder des Fluidauslasses 3 radial innenliegend von dem Konnektor 4 zwischen dem Konnektor 4 und dem jeweiligen Anschlussstück 8, 9 angeordnet. D.h. dieser O-Ring dichtet als Radialdichtung einen Ringspalt zwischen dem Anschlussstück 8, 9 und dem buchsenförmigen Konnektor 4 ab. Der zweite O-Ring 10 liegt abdichtend zwischen der Außenfläche (axiale Stirnseite) des Konzentrat-/Aufnahmebehälters 1a und einem radialen Flanschabschnitt 8a, 9a des jeweiligen Anschlussstücks 8, 9 und dichtet somit als Axialdichtung den Axialspalt zwischen der Stirnseite des Aufnahmebehälters 1a und dem Flansch/Kragen 8a, 9a des Anschlussstücks 8, 9 ab.

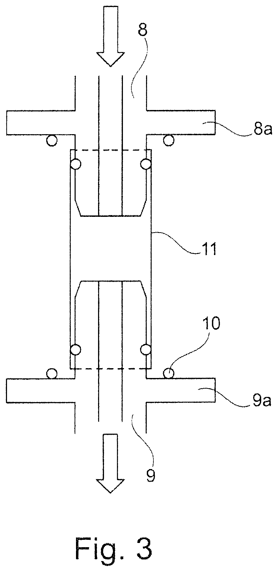

Fig. 3 zeigt ein Verbindungsstück 11, welches während der Reinigung/Desinfektion der extrakorporalen Blutbehandlungsmaschine in ein erfindungsgemäßes (Leitungs-) System für eine extrakorporale Blutbehandlungsmaschine zur Herstellung beispielsweise einer Pufferlösung eingesetzt ist. Das Verbindungsstück 11 ist hierfür als Hohlzylinder ausgebildet und wird an Stelle des Konzentratbehälters/Kartusche 1 auf die Anschlussstücke 8, 9 des erfindungsgemäßen (Leitungs-) Systems aufgesteckt. Zu diesem Zweck wird an den beiden axial beabstandeten Enden des Verbindungsstücks 11 jeweils ein Konnektor simuliert, der dafür ausgelegt ist, die Anschlussstücke des Maschinenseitigen (Leitungs-) Systems aufzunehmen.

Ist das Verbindungsstück 11 axial endseitig auf die Anschlussstücke 8, 9 aufgesteckt, um diese kurz zu schließen, so kann beispielsweise eine Reinigungsflüssigkeit in Fluidflussrichtung (dickgedruckter Pfeil) durch das erfindungsgemäße (Leitungs-) System für eine extrakorporale Blutbehandlungsmaschine geleitet werden. Die Abdichtung der Verbindung zwischen dem Verbindungsstück 11 und den Anschlussstücken 8, 9 erfolgt jeweils über einen O-Ring 10, der als Ringdichtung zwischen der radialen Innenwand des Hohlzylinders des Verbindungsstücks 11 und der radialen Außenwand des in das Verbindungsstück 11 eingeführten Anschlussstücks 8, 9 liegt, um den dazwischen sich ausbildenden Ringspalt abzudichten.

## Patentansprüche

1. Kartuschenförmiger Konzentratbehälter (1) für eine extrakorporale Blutbehandlungsmaschine, mit
einem Aufnahmebehälter (1a),
einem Fluideinlass (2) an einem ersten axialen Ende des Aufnahmebehälters (1a) und
einem Fluidauslass (3) an einem zweiten axialen Ende des Aufnahmebehälters (1), sowie
mindestens einem den Fluideinlass (2) und/oder den Fluidauslass (3) bildenden Konnektor (4), der zur Kopplung mit einem Anschlussstück (8, 9) wenigstens einer Fluideinlass- und/oder Fluidauslassleitung der extrakorporalen Blutbehandlungsmaschine ausgelegt ist,
**dadurch gekennzeichnet, dass**
der mindestens eine Konnektor (4), zur Aufnahme des zugehörigen Anschlussstücks (8, 9) in sich, zurückweichend, eingebuchtet und in den Aufnahmebehälter (1a) hineinragend ausgebildet ist und vollständig innerhalb des Aufnahmebehälters (1a) angeordnet ist.

2. Kartuschenförmiger Konzentratbehälter (1) nach Anspruch 1, **dadurch gekennzeichnet, dass** das zweite axiale Ende des Aufnahmebehälters (1) in Form eines Kugelschalensegments oder eines Kegelstumpfs ausgebildet ist.

3. Kartuschenförmiger Konzentratbehälter (1) nach einem der Ansprüche 1 bis 2, weiterhin **gekennzeichnet durch** ein Fluidlenkungsmittel (7), welches in Fluidflussrichtung mindestens jenem Konnektor (4) zur Aufnahme des Anschlussstücks (8) der Fluideinlassleitung nachgeschaltet ist.

4. Kartuschenförmiger Konzentratbehälter (1) nach Anspruch 3, **dadurch gekennzeichnet, dass** das Fluidlenkungsmittel (7) Strukturen zur gezielten Lenkung des einströmenden Fluids aufweist.

5. Kartuschenförmiger Konzentratbehälter (1) nach Anspruch 4, **dadurch gekennzeichnet, dass** das Fluidlenkungsmittel (7) und der Konnektor (4), welchem das Fluidlenkungsmittel (7) in Fluidflussrichtung nachgeschaltet ist, ein integrales, einstückiges Bauteil bilden.

6. Kartuschenförmiger Konzentratbehälter (1) nach Anspruch 4 oder 5, **dadurch gekennzeichnet, dass** das Fluidlenkungsmittel (7) eine Aufprallscheibe ist, auf die in den Aufnahmebehälter (1) einströmendes Fluid aufprallt, wodurch das Fluid radial gleichmäßig auf das in dem Konzentratbehälter (1) eingelagerte Konzentrat (5) verteilt wird.

7. Kartuschenförmiger Konzentratbehälter (1) nach einem der Ansprüche 4 bis 6, **dadurch gekennzeichnet, dass** das Fluidlenkungsmittel (7) drehbar über ein turbinenartiges Flügelrad gelagert ist.

8. Kartuschenförmiger Konzentratbehälter (1) nach einem der Ansprüche 4 bis 6, **dadurch gekennzeichnet, dass** das Fluidlenkungsmittel (7) als Sieb ausgebildet ist.

9. Leitungs-System für eine extrakorporale Blutbehandlungsmaschine zur Herstellung eines Fließmittels, mit:
einem Konzentratbehälter (1) nach einem der Ansprüche 1 bis 8, sowie mindestens einem Anschlussstück (8) zum Fluidverbinden des Konzentratbehälters (1) mit einer Fluideinlassleitung des Leitungs-Systems, und
mindestens einem Anschlussstück (9) zum Fluidverbinden des Konzentratbehälters (1) mit einer Fluidauslassleitung der Leitungs-Systems,
**dadurch gekennzeichnet, dass**
mindestens eines der Anschlussstücke (8, 9) hervorstehend und/oder hervorragend ausgebildet und dazu ausgelegt ist, in den entsprechenden zurückweichenden und/oder eingebuchteten und innerhalb des Aufnahmebehälters (1a) angeordneten Konnektors (4) des Konzentratbehälters (1) einzugreifen.

## Claims

1. Cartridge-shaped concentrate container (1) for an extracorporeal blood treatment machine, comprising
a receptacle (1a),
a fluid inlet (2) on a first axial end of the receptacle (1a) and
a fluid outlet (3) on a second axial end of the receptacle (1a) and
at least one connector (4) forming the fluid inlet (2) and/or the fluid outlet (3) which is designed to couple with a coupling fitting (8, 9) of at least a fluid inlet and/or fluid outlet conduit of the extracorporeal blood treatment machine,
**characterized in that**
the at least one connector (4), for holding the respective coupling fitting (8, 9) in it, is configured to be recessed and indented and protrudes into the receptacle (1a) and is arranged entirely inside the receptacle (1a).

2. Cartridge-shaped concentrate container (1) according to Claim 1,
**characterized in that** the second axial end of the receptacle (1a) is configured in the shape of a spherical shell segment or a truncated cone.

3. Cartridge-shaped concentrate container (1) according to one of the Claims 1 to 2, further **characterized by** a fluid ducting device (7) which is positioned downstream in the direction of flow of the fluid from at least said connector (4) for holding the coupling fitting (8) of the fluid inlet conduit.

4. Cartridge-shaped concentrate container (1) according to Claim 3,
**characterized in that** the fluid ducting device (7) comprises structures for the selective deflection of the flowing fluid.

5. Cartridge-shaped concentrate container (1) according to Claim 4,
**characterized in that** the connector (4) and the fluid ducting device (7), which is downstream in the direction of flow of the fluid from the connector (4), form a single, integral component.

6. Cartridge-shaped concentrate container (1) according to Claim 4 or 5,
**characterized in that** the fluid ducting device (7) is an impact disc against which fluid flowing into the receptacle (1a) impacts, whereby the fluid is spread in a radially even manner onto the concentrate (5) deposited in the concentrate container (1).

7. Cartridge-shaped concentrate container (1) according to one of Claims 4 to 6,
**characterized in that** the fluid ducting device (7) is pivoted by means of a turbine-like impeller wheel.

8. Cartridge-shaped concentrate container (1) according to one of Claims 4 to 6,
**characterized in that** the fluid ducting device (7) is configured as a sieve.

9. Conduit system for an extracorporeal blood treatment machine for producing a flow agent, comprising:
a concentrate container (1) according to one of Claims 1 to 8, and
at least one coupling fitting (8) to form a fluid connection between the concentrate container (1) and a fluid inlet conduit of the conduit system, and
at least one coupling fitting (9) to form a fluid connection between the concentrate container (1) and a fluid outlet conduit of the conduit system,
**characterized in that**
at least one of the coupling fittings (8, 9) is salient and/or protruding in shape and is designed to engage with the respective recessed and/or indented connector (4), which is positioned inside the receptacle (1a), of the concentrate container (1).

## Revendications

1. Récipient pour produit concentré (1) en forme de cartouche, destiné à une machine de traitement extracorporel du sang, avec
- un récipient récepteur (1a),
- une entrée de fluide (2) à une première extrémité axiale du récipient récepteur (1a) et
- une sortie de fluide (3) à une deuxième extrémité axiale du récipient récepteur (1), ainsi que
- au moins un connecteur (4) qui forme l'entrée de fluide (2) et/ou la sortie de fluide (3) et qui est conçu pour le couplage avec une pièce de raccordement (8, 9) d'au moins une conduite d'entrée de fluide et/ou de sortie de fluide de la machine de traitement extracorporel du sang,
**caractérisé en ce que,** pour recevoir la pièce de raccordement (8, 9) associée, l'au moins un connecteur (4) est réalisé rentrant en lui-même, en retrait, et pénétrant dans le récipient récepteur (1a) et il est agencé entièrement à l'intérieur du récipient récepteur (1a).

2. Récipient pour produit concentré (1) en forme de cartouche selon la revendication 1,
**caractérisé en ce que** la deuxième extrémité axiale du récipient récepteur (1) est réalisée sous la forme d'un segment de coque sphérique ou d'un cône tronqué.

3. Récipient pour produit concentré (1) en forme de cartouche selon l'une quelconque des revendications 1 à 2, **caractérisé en outre par** un moyen de guidage de fluide (7) qui est placé en aval, dans le sens d'écoulement du fluide, au moins du connecteur (4) destiné à recevoir la pièce de raccordement (8) de la conduite d'entrée de fluide.

4. Récipient pour produit concentré (1) en forme de cartouche selon la revendication 3,
**caractérisé en ce que** le moyen de guidage de fluide (7) comporte des structures destinées à guider de manière ciblée le fluide affluant.

5. Récipient pour produit concentré (1) en forme de cartouche selon la revendication 4, **caractérisé en ce que** le moyen de guidage de fluide (7) et le connecteur (4) en aval duquel le moyen de guidage de fluide (7) est placé dans le sens d'écoulement du fluide forment une partie intégrée d'un seul tenant.

6. Récipient pour produit concentré (1) en forme de cartouche selon la revendication 4 ou 5, **caractérisé en ce que** le moyen de guidage de fluide (7) est un disque de rebond sur lequel rebondit le fluide affluant dans le récipient récepteur (1), répartissant ainsi le fluide uniformément de façon radiale sur le produit concentré (5) stocké dans le récipient pour produit concentré (1).

7. Récipient pour produit concentré (1) en forme de cartouche selon l'une quelconque des revendications 4 à 6, **caractérisé en ce que** le moyen de guidage de fluide (7) est monté de manière à pouvoir tourner par l'intermédiaire d'une roue à ailettes du type turbine.

8. Récipient pour produit concentré (1) en forme de cartouche selon l'une quelconque des revendications 4 à 6, **caractérisé en ce que** le moyen de guidage de fluide (7) est réalisé sous forme de crible.

9. Système de conduite destiné à une machine de traitement extracorporel du sang en vue de la fabrication d'un fluidifiant, avec :
un récipient pour produit concentré (1) selon l'une quelconque des revendications 1 à 8, ainsi que
au moins une pièce de raccordement (8) destinée à la liaison fluidique du récipient pour produit concentré (1) avec une conduite d'entrée de fluide du système de conduite, et
au moins une pièce de raccordement (9) destinée à la liaison fluidique du récipient pour produit concentré (1) avec une conduite de sortie de fluide du système de conduite,
**caractérisé en ce que**
au moins l'une des pièces de raccordement (8, 9) est réalisée de manière à dépasser et/ou à faire saillie et est conçue pour pénétrer dans le connecteur (4) correspondant, en retrait et/ou rentrant et agencé à l'intérieur du récipient récepteur (la), du récipient pour produit concentré (1).
